# EUROPÄISCHE PATENTSCHRIFT

(11) **EP 2 152 173 B1**
(45) Veröffentlichungstag und Bekanntmachung des Hinweises auf die Patenterteilung: **30.09.2015**
(21) Anmeldenummer: 08758655.8
(22) Anmeldetag: 20.05.2008
(51) Int. Cl.: A61B 17/17, A61B 17/86, A61B 17/72

(54) **VERRIEGELUNGSMARKNAGEL**
LOCKING INTRAMEDULLARY NAIL
CLOU DE VERROUILLAGE INTRAMÉDULLAIRE

(30) Priorität: 23.05.2007 DE 102007023891; 24.10.2007 DE 102007051136; 18.03.2008 DE 102008014697
(43) Veröffentlichungstag der Anmeldung: 17.02.2010
(73) Patentinhaber: Schreiber, Ulrich, D-80639 München (DE)
(72) Erfinder: Schreiber, Ulrich, D-80639 München (DE)
(74) Vertreter: Bobbert & Partner Patentanwälte PartmbB
(86) Internationale Anmeldenummer: PCT/EP2008/004048
(87) Internationale Veröffentlichungsnummer: WO 2008/141805

(56) Entgegenhaltungen:
- EP-A- 1 839 608
- WO-A-2004/096067
- US-A1- 2002 173 792
- US-A1- 2005 234 457

## Beschreibung

Die Erfindung bezieht sich auf einen Verriegelungsmarknagel zum Einbringen in den Markraum eines Röhrenknochens gemäß Anspruch 1.

Marknägel und speziell verriegelbare Marknägel sind bekannte Hilfsmittel zur Versorgung von Brüchen langer Röhrenknochen, die in den Markraum des lädierten Knochens eingebracht werden, um die Knochenläsion mechanisch zu überbrücken. Bei den so genannten Verriegelungsmarknägeln dienen Verriegelungsschrauben, welche Knochen und Verriegelungsnagel überbrückend und/ oder verbindend, insbesondere quer durch oder in den Knochen und den Verriegelungsnagel oder Abschnitte hiervon, eingebracht sind, dazu, die Verbindung zwischen Knochen und Verriegelungsnagel gegen Verschiebung, insbesondere des Verriegelungsnagels, in Richtung der Knochenachse sowie gegen Rotation, insbesondere des Verriegelungsnagels; um die Knochenachse zu sichern.

Bei bisher üblichen Marknägeln werden die Verriegelungsschrauben in diskreten Öffnungen des Marknagels in einer festen Position platziert. Üblicherweise sind hierbei die Verriegelungsschrauben in dem Marknagel im Wesentlichen radial ausgerichtet, können aber, wie beispielsweise am proximalen Oberschenkel, auch in einem vorgegebenen Neigungswinkel zur Längsachse des Marknagels ausgerichtet sein.

Das Setzen der Verriegelungsschrauben in dem im Röhrenknochen angeordneten Marknagel setzt hierbei eine hohe Erfahrung des Operateurs voraus, um den Marknagel exakt zu platzieren und zu fixieren. Der Operateur hat praktisch keine Möglichkeit, auch geringfügige Fehlausrichtungen der Verriegelungsschraube während der Operation zu korrigieren.

In keinem Falle ist es bisher möglich, während der Operation die Lage und Winkel der Bohrungen für die Verriegelungsschrauben und deren Lage an die individuelle anatomische und sich aus der Verletzung ergebende Situation während der Operation anzupassen. Es wäre jedoch in vielen Fällen vorteilhaft, den Winkel der den Marknagel durchdringenden Verriegelungsschrauben während der Operation variieren zu können, um Frakturfragmente zu repositionieren beziehungsweise anatomisch korrekt zu adaptieren. Anschließend sollten die Verriegelungsschrauben in ihrer gewählten Position winkelstabil zu verblocken sein.

Ein Verriegelungsmarknagel mit den Merkmalen des Oberbegriffs des Anspruchs 1 ist aus der US 2005/234457 A1 bekannt.

Aufgabe der vorliegenden Erfindung ist es, einen weiteren Verriegelungsmarknagel (im Folgenden auch als Marknagel bezeichnet) anzugeben.

Die erfindungsgemäße Aufgabe wird mit einem Marknagel mit den Merkmalen des Anspruchs 1 gelöst.

Weitere Ausgestaltungen der Erfindung sind jeweils Gegenstand der Unteransprüche.

Demgemäß ist im Bereich zumindest einer Öffnung des Marknagels eine zumindest in einer Raumrichtung - bspw. in einer Richtung senkrecht zu der Längsachse des Knochen- oder Marknagelschaftes- und/ oder in einer Ebene, insbesondere einer die zuvor genannte Richtung enthaltende Ebene, ausrichtbare, oder bewegbare oder verschiebbare oder verschwenkbare Hülse in dem Schaft und/ oder am Schaft angeordnet und/ oder gelagert. Die Hülse weist eine Öffnung oder Bohrung zur Aufnahme einer Verriegelungsschraube oder mehrere solcher oder anderer Verriegelungseinrichtungen auf. Nach Einsetzen der Verriegelungsschraube(n) in den Schaft wird diese Hülse wiederum verriegelt bzw. blockiert bzw. arretiert. Weiterhin ist ein Stützkörper zwischen zwei aufeinander folgenden Hülsen in oder am Verriegelungsmarknagel vorgesehen. Der Stützkörper liegt an den beiden Hülsen an. Die Anordnung aus den Hülsen und dem Stützkörper ist durch zumindest eine in Längsrichtung des Verriegelungsmarknagels wirkende Klemmschraube zusammenpressbar und/ oder blockierbar und/ oder arretierbar.

Dabei kann die Verschwenkbarkeit der Hülse und ihre Nicht-Verschwenkbarkeit nach Verriegelung oder Blockung an einer zugelassenen oder unterbundenen Bewegung, insbesondere Neigbarkeit oder Drehbarkeit, einer in Bezug auf die Hülse festlegbaren virtuellen Achse oder Geraden bestimmt/ festgemacht werden. Diese Achse kann eine Längsachse oder eine Symmetrieachse der Öffnung oder Bohrung der Hülse sein.

Die Hülse kann erfindungsgemäß eine Einrichtung mit wenigstens einer vorgefertigten Durchgangsöffnung für die Verriegelungsschraube sein.

Nicht erfindungsgemäß umfasst "Hülse" auch eine Masse, insbesondere eine aushärtbare Masse wie Knochenzement, welche zu einem frühen ersten Zeitpunkt ein Ausrichten des oder der durch die hindurch geführte(n) Verriegelungsschraube(n) erlaubt, nicht mehr aber zu einem späteren zweiten Zeitpunkt. Auch eine Arretierung mittels eines Materials, welches bei unterschiedlichen Umgebungstemperaturen unterschiedliche Formen einnimmt und sich bei Wärme ausdehnen oder zusammenziehen kann, wird, wenn der Arretiereffekt durch diese Materialeigenschaft bewirkt oder begünstigt wird, erfindungsgemäß unter der Arretiereinrichtung oder Hülse verstanden.

Die Hülse kann zum Beispiel eine Walze mit einer Walzenachse, welche bspw. senkrecht zu der Längsachse des Schafts des Verriegelungsnagels steht, sein, ist jedoch bevorzugt kugelförmig, sodass sie in allen Raumachsen frei schwenkbar in dem Schaft gelagert ist.

Die Hülse kann aus mehreren Teilen und/oder mehreren Materalien zusammengesetzt sein (Composit) (nicht Teil der Erfindung). Beispielsweise kann ein Kunststoffring als Teil der Hülse zum Einsatz kommen. Er kann ein ungewolltes Herausdrehen der Vernegelungsschraube(n) vorteilhaft verhindern.

Unter der "Bohrung", welche in der Hülse vorgesehen ist, wird erfindungsgemäß eine Bohrung verstanden, wie sie dem Fachmann bekannt ist. Sie ist erfindungsgemäß eine Durchgangsöffnung zur Aufnahme der Verriegelungsschraube oder eines Abschnitts hiervon vorgesehen sein, welche der Fachmann nicht als eine Bohrung ansieht. Eine solche Durchgangsöffnung kann bspw. gegossen oder bei einem Gießvorgang (mit-)entstanden sein. Beim Gießen muss - anders als beim Erzeugen einer Bohrung kein Hülsenmaterial abgetragen oder beseitigt werden. Eine solche nicht gebohrte Durchgangsöffnung kann ferner zu Zusammenbringen mehrerer Teilhülsen mit entsprechenden Aussparungen zustande kommen.

Unter einer Verriegelung wird bspw. ein Verhindern einer Bewegung - z.B. des Marknagels - in einer Längsachse hiervon oder um seine Längsachse herum nach Abschluss dessen Einbringen in den Knochen verstanden. Es wird im Zusammenhang mit der vorliegenden Erfindung jedoch auch ein Fixieren von Frakturfragmenten oder anderen Knochenabschnitten verstanden. Unter Fixieren wird auch der Versuch verstanden, den Abstand zwischen Knochenabschnitten oder -fragmenten zu bewahren. Ein Sintern, insbesondere ein ärztlich gewolltes Sintern, der Knochenabschnitte, bei welchem sich der Abstand trotz Verriegelung in Begrenztem Umfang verändern darf, ist hierbei dem Fixieren nicht abträglich. Daher kann eine Verriegelungsschraube oder -einrichtung im Sinne der vorliegenden Erfindung sowohl eine Einrichtung zum Fixieren des Marknagels im Knochen, als auch eine Einrichtung zum Sichern von wenigstens zwei Knochen öder -abschnitten oder -fragmenten relativ zueinander sein. Mit einer Konstruktion eines Marknagels gemäß der Erfindung können beim Einsetzen einer Verriegelungsschraube in den Marknagel geringe Fehlstellungen der Verriegelungsschraube quasi automatisch ausgeglichen werden. Zudem hat der Operateur, insbesondere bei einer kugelförmigen Hülse, die in drei Freiheitsgraden ausrichtbar ist, die Möglichkeit, noch während der Operation den Winkel der den Marknagel durchdringenden Verriegelungsschraube(n) bspw. zum Schaft und/ oder die Ausrichtung der Verriegelungsschraube(n) im Raum variieren zu können. Hierdurch hat der Operateur eine Möglichkeit, die Frakturfragmente des Knochens oder des Gelenkes präzise zu repositionieren beziehungsweise anatomisch korrekt zu adaptieren, bzw. zu komprimieren.

Die Verriegelung der Hülse mit der Verriegelungsschraube beziehungsweise die Lagefixierung oder Blockierung der Hülse kann auf mehrere Arten erfolgen. Die Hülse, die bevorzugt aus Kunststoff ist, und die unabhängig hiervon ähnlich wie bekannte Dübel mit einem Schlitz versehen sein kann, kann beispielsweise durch eine konische oder anders geeignete Ausbildung der Verriegelungsschraube aufgeweitet und fixiert werden. Ebenso ist es möglich, diese Lagefixierung der Hülse mit der Verriegelungsschraube durch eine zum Schaft des Marknagels bspw. koaxial und/oder im Wesentlichen parallel angeordnete Klemmschraube zu erreichen. Eine solche Klemmschraube kann dabei auch bspw. als ein Passstift oder eine verriegelbare Einrichtung oder jede andere Einrichtung ausgestaltet sein bzw. durch diese ersetzt sein, welche eine Druck- oder Zugwirkung auf die Hülse ausüben zu deren Inmobilisierung im Raum oder in wenigstens einer Ebene kann. Falls mehrere Hülsen in dem Marknagel vorgesehen sind, können diese fixiert, insbesondere positions-, und/oder winkel- und/oder lagefixiert, werden, indem bspw. zwischen den einzelnen Hülsen Zwischenkörper vorgesehen sind, wobei dann die einzelnen Hülsen durch eine Einrichtung, z. B. durch eine Klemmschraube, fixiert werden.

Die Zwischenkörper können massiv oder mit wenigstens einem Hohlraum ausgestaltet sein. Durch die Holräume kann sich eine größere Deformität der Zwischenkörper ergeben, was wiederum die Reibung und somit die Winkelstabilität erhöht.

Die Zwischenkörper können so ausgeführt sein, dass sie ähnlich einer Verzahnung eine formschlüssige Verbindung mit der Hülse eingehen können, um so bei der Verblockung einen erhöhten Reibwiderstand leisten zu können und damit die Winkelstabilität zu erhöhen.

Der erfindungsgemäße Marknagel kann in einem modularen Aufbau derart ausgestaltet sein, dass der Marknagel eine koaxiale Bohrung oder Öffnung aufweist, in welche ein Einsatz mit wenigstens einer Hülse oder eine zusammenhängende Einheit von Hülsen und Zwischenkörpern eingebracht werden kann. Diese Ausgestaltung erlaubt vorteilhaft, dass vor dem Einbringen des Einsatzes die Öffnung für eine Kanülierung nutzbar ist mit den für den Fachmann im Zusammenhang mit der Kanülierung bekannten Vorteilen.

Die Positionierung der Verriegelungsschrauben wird durch eine Vorrichtung erleichtert, indem mit einem Ende des Marknagels ein gekrümmter Führungsbogen lösbar verbunden ist (nicht Teil der Erfindung). Auf diesem Führungsbogen gleitet eine Schablone mit einer auf den Marknagel auszurichtenden, insbesondere geradlinigen, Zieleinrichtung, die in einer, die Mittelebene des Marknagels enthaltenden Ebene verschiebbar ist, wobei die Zieleinrichtung ausgebildet ist, um eine Verriegelungsschraube oder ein Instrument zum Einbringen der Verriegelungseinrichtung, z. B. zum Einschrauben der Verriegelungsschraube, in der Hülse auszunehmen.

Der Führungsbogen verläuft vorzugsweise längs einer Bahn, bspw. eines Bogens, insbesondere eines Kreisbogens und weist insbesondere eine in seiner Längsrichtung verlaufende, insbesondere im Wesentlichen mittig vorgesehene, Führungsbahn auf, die den Führungsbogen durchstößt und auf den Marknagel ausgerichtet ist. In dieser Führungsbahn gleitet eine Schablone mit der durch die Führungsbahn hindurch gerichteten Zieleinrichtung, die insbesondere eine auf den Marknagel gerichtete und die Schablone durchstoßende Öffnung aufweist. Die Öffnung kann noch durch ein koaxiales Führungsrohr verlängert werden.

Der Führungsbogen kann bogenförmig verlaufen, jedoch kann er auch bogenförmige oder anders gekrümmte Abschnitte neben geraden Abschnitten aufweisen.
Der Führungsbogen kann auch nur aus geraden Abschnitten bestehen, ohne jeden gekrümmten Abschnitt.

Der Führungsbogen kann bei der Explantation durch die oben genannten Ankopplungsmechanismen am Marknagel derart fixiert werden, dass durch den Führungsbogen die Entriahme (Herausschrauben) der Verriegelungsschrauben erleichtert wird und damit die Notwendigkeit der intensiven Strahlenbelastung minimiert werden kann. Durch die genaue Positionierbarkeit der Instrumente mit Hilfe des Führungsbogens wird die Auffindbarkeit der Schraubenköpfe der Verriegelungsschrauben erleichtert. Eine minimalinvasive Implantatentfernung ohne Strahlenbelastung kann so durchgeführt werden.

Durch die in dem Schaft beweglich gelagerte Hülse kann die Verriegelungsschraube zwanglos in die Bohrung der Hülse treffen, wobei, insbesondere wenn mehrere Hülsen zur Fixierung einzelner Frakturelemente vorgesehen sind, an dem Führungsbogen Markierungen oder Anschläge für die Schablone vorgesehen sein können. Rasterungen können in axialer und/ rotatorischer Richtung am Führungsbogen vorgesehen sein.

Hülsen können dabei anstelle mit Verriegelungsschrauben auch mit Platzhalter besetzt werden. Dies hat den Vorteil, dass eine nicht mit einer Verriegelungsschraube belegte Hülse mittels des Platzhalters eine gewünschten Kraftfluss über sie hindurch erlaubt, wenn nach Abschluss des Einbringens des Marknagels in den Knochen die axiale Verspannung für eine winkelstabile Sicherung genutzt wird.

Es wird darauf hingewiesen, dass der erfindungsgemäße Marknagel zusammen mit einer oder mehreren Verriegelungsschrauben verwendet werden kann. Daher ist die Verwendung der Begriffe "Verriegelungsschraube" und "Verriegelungsschrauben" in der vorliegenden Beschreibung austauschbar: Wo von "Verriegelungsschraube" die Rede ist, sind auch "Vernegelungsschrauben" zu verstehen und umgekehrt, wo immer dies technisch möglich ist. Dabei steht der Begriff "Verriegelungsschraube" für eine Verriegelungseinrichtung allgemein.

Daher kann die Verriegelungsschraube ferner als Verriegelungsstift ausgestaltet sein. Ein Gewinde ist erfindungsgemäß nicht erforderlich. Das Vorsehen einer Passung oder sonstigen Einrichtung zum Erhöhen der Reibung zwischen Hülse und Verriegelungsschraube kann vorteilhaft ein mit der Zeit erfolgendes, unerwünschtes Heraustreten der Verriegelungsschraube entlang seiner Längsrichtung aus der Hülse verhindern. Diese Einrichtungen zum Erhöhen der Reibung können dabei über lediglich einem Abschnitt (bzw. entlang des Abschnitts) der Hülse und/ oder der Verriegelungsschraube oder deren gesamte Erstreckung vorgesehen sein.

In manchen erfindungsgemäßen Ausführungsformen wird zum Versorgen eines Röhrenknochens ein verriegelbarer Marknagel verwendet. Dieser Knochennagel kann einen Schaft mit einer Längsachse aufweisen, wobei insbesondere der Schaft wenigstens eine Öffnung zum Einbringen - z. B zum Hinein- oder Hindurchführen - von wenigstens einer Verriegelungseinrichtung aufweist. Die Verriegelungseinrichtung kann dabei - wie in einer bevorzugten erfindungsgemäßen Ausführungsform vorgesehen - insbesondere als eine Verriegelungsschraube oder ein Verriegelungsstift ausgestaltet sein kann

Der Knochennagel weist wenigstens eine Aufnahmeeinrichtung - wie z. B. der oben beschriebenen Hülse - zum Aufnehmen wenigstens eines Abschnitts der Verriegelungseinrichtung auf, wobei die Aufnahmeeinrichtung in wenigstens einer ihrer Raumachsen in Bezug zur Längsachse des Knochens und /oder des Marknagels ausrichtbar, insbesondere verschwenkbar, angeordnet ist, und wobei deren Ausrichtbarkeit in Bezug zur Längsachse unterbindbar ist, insbesondere mittels einer Arretiereinrichtung. Unter Ausrichtung ist hierbei auch ein Ausrichten der Verriegelungseinrichtung in Bezug auf die Aufnahmeeinrichtung und/ oder den Knochen und/ oder den Marknagel zu verstehen.

Unter dem "Unterbinden" wird erfindungsgemäß ein Beenden der Ausrichtbarkeit der in die Aufnahmeeinrichtung eingeführten Verriegelungseinrichtung, z. B. einer Verriegelungsschraube, im Raum, wenigstens in zumindest einer Raumrichtung oder einer Drehrichtung, verstanden. Dies kann durch ein Blockieren, ein Arretieren, ein Aushärten, ein Verriegeln oder dergleichen der Aufnahmeeinrichtung geschehen. Hierzu kann eine Einrichtung zum Unterbinden wie einer oben beschriebenen Klemmschraube vorgesehen sein. Mit Unterbinden kann jedoch auch eine Wirkung oder Eigenschaft gemeint sein, die einer der Strukturen innewohnt. Als Beispiel wird hier die Aushärtbarkeit einer mittels Zement ausgestalteten Aufnahmeinrichtung genannt.

Ein weiteres Beispiel, wie die Ausrichtbarkeit der Aufnahmeeinrichtung unterbunden werden kann (nicht Teil der Erfindung), ist das Vorsehen einer Einrichtung zur Erhöhung der Reibung zwischen Verriegelungseinrichtung und Knochennagel, wobei ein Einbringen der Verriegelungseinrichtung (bspw. durch Schrauben, Einschlagen, Einschieben oder dergleichen) in die Aufnahmeeinrichtung die Ausrichtbarkeit der Aufnahmeeinrichtung zunehmend oder schlagartig beendet. Dies kann durch ein Aufspreizen der Aufnahmeeinrichtung mittels des bspw. konisch zulaufenden Schafts der Verriegelungseinrichtung bewerkstelligt werden. Durch ein solches Aufspreizen oder auch nur eine entsprechende Druckerhöhung mit Verformung einer äußeren Kontur der Aufnahmeinrichtung oder wenigstens Erhöhen des Drucks der Aufnahmeeinrichtung auf ihre Umgebung kann die Aufnahmeeinrichtung gegen eine weitere Struktur des Marknagels derart gedrückt werden, dass die Aufnahmestruktur gegenüber dem Marknagel in ihrer Bewegungsfreiheit und insbesondere in ihrer Ausrichtbarkeit blockiert ist. Sie kann also allein durch das Eintreiben der Verriegelungseinrichtung in sie hinein arretiert werden.

Die Verriegelungseinrichtung (bspw. in Stift- oder Schraubenform) kann dabei derart ausgestaltet sein, dass erst das Einbringen ihres zuletzt in die Aufnahmeeinrichtung einzubringenden Abschnitts die Blockierung bewirkt. Es kann die Verriegelungseinrichtung für eine solche Blockierung vorbereitet sein. Die Aufnahmeeinrichtung kann ebenfalls entsprechend ausgestaltet sein. Zudem können sowohl die Verriegelungseinrichtung als auch die Aufhahmeeinrichtung zum Erzielen der Blockierung bzw. Verrieglung ausgestaltet sein.

Alle erfindungsgemäßen Gegenstände können aus einer Typ II anodisierten Titanlegierung (Ti6A14V) für verbesserte biomechanische und biomedizinische Leistungen gefertigt sein.

Mittels der vorliegenden Offenbarung können mit Hilfe der Zieleinrichtung bzw. der Vorrichtung mit dem Führungsbogen vor dem Schraubensetzen auch Bohrer, Spick- oder Führungsdrähte oder auch ein anderes Werkzeug vorteilhaft ausgerichtet, positioniert, und/ oder geführt eingebracht werden.

Zudem kann auch eine zu einem späteren Zeitpunkt ggf. erforderliche "Metallentfernung", also ein Auffinden und Herausdrehen von verwendeten Schrauben mit Hilfe des Zielbügels erleichtert werden. Auch der Marknagel kann unter Verwendung der Vorrichtung in axialer Richtung (wie auch der Zielbügel) angekoppelt werden, wodurch ein Entfernen des Implantates aus dem Markraum ermöglicht oder erleichtert wird.

Die Erfindung wird im Folgenden anhand der Zeichnungen näher erläutert. In dieser gilt:
Figur 1 zeigt einen Marknagel gemäß der Erfindung mit einer in dem Schaft des Marknagels verschwenkbar angeordneten Hülse, die eine Verriegelungsschraube zum Fixieren des Marknagels aufnimmt;
Figur 2 zeigt eine schematische Darstellung einer Verriegelungsschraube und einer Hülse zur Verriegelung des Marknagels in einem Knochen;
Figur 3 zeigt einen in den Markraum eines Knochens implantierten Marknagel mit einer Klemmschraube zur lagestabilen Fixierung des Marknagels;
Figur 4 zeigt einen Querschnitt durch einen Marknagel mit zwei jeweils zur Aufnahme einer Verriegelungsschraube ausgebildeten Hülsen, die miteinander durch einen Zwischenkörper und eine Klemmschraube fixiert sind; und
Figur 5 zeigt eine perspektivische Ansicht einer nicht erfindungsgemäßen Vorrichtung zum Erleichtern der Fixierung eines Marknagels in einem Knochen oder einen Gelenk.

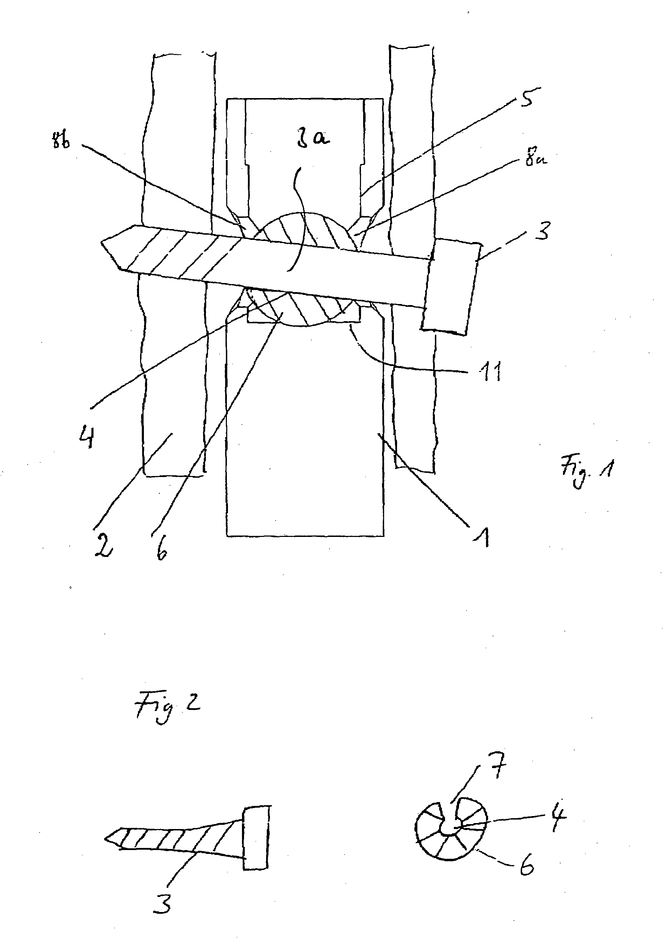

In Figur 1 ist ein Marknagel 1 dargestellt, der in den Markraum eines Knochens 2 implantiert ist. In den Marknagel 1 ist im Bereich, in dem eine Verriegelungsschraube 3 platziert werden soll, in einem Schaft 5 des Marknagels 1 eine kugelförmige Hülse 6 eingebracht, die zur Aufnahme der Verriegelungsschraube 3 eine Bohrung 4 aufweist. Der Schaft 5 weist im Bereich der Hülse 6 zwei gegenüber liegende Durchbrüche 8a, 8b auf, durch welche die Verriegelungsschraube 3 hindurch geführt werden kann, wobei der Abschnitt 3a in der Hülse 6 zum Liegen kommt. Die Hülse 6 liegt auf einer Sitzfläche 11 im Marknagel 1 auf und ist frei drehbar im Schaft 5 gelagert. Hierdurch kann die Verriegelungsschraube 3 während der Operation in einem durch die Durchbrüche 8a, 8b im Schaft 5 begrenzten Winkelbereich in beliebiger Neigung durch die Bohrung 4 im Marknagel 1 hindurch getrieben werden. Auch werden leichte Fehlstellungen der Hülse 6 oder der Verriegelungsschraube 3 kompensiert, sodass die Verriegelungsschraube 3 stets in die Bohrung 4 der Hülse 6 eingreift.

Durch die Winkelflexibilität bzw. -ausrichtbarkeit beim Einbringen der Verriegelungsschraube(n) 3 besteht die Möglichkeit, den Marknagel 1 während der Implantation an die individuelle anatomische beziehungsweise pathologische Situation des Patienten optimal anzupassen.

Die Verriegelung der Hülse 6 in dem Schaft 5 kann auf unterschiedliche Weise erfolgen.

Ein Beispiel zeigt die Figur 2. Die Verriegelungsschraube 3 weist hierbei einen teilweise oder vollständig konisch ausgebildeten Schraubenschaft 3a auf; die Hülse 6 mit ihrer Öffnung 4 ist ähnlich einem Dübel mit einem Schlitz 7 versehen. Die Hülse 6 wird dann beim Eindrehen der Vernegelungsschraube 3 aufgeweitet und im Schaft 5 des Marknagels 1 verspreizt. Die Hülse legt sich dann an entsprechende Anschläge, zum Beispiel die Ränder der Durchbrüche 8a und 8b an. Durch diese kraftschlüssige Fixierung der kugelförmigen Hülse 6 in Schaft 5 wird die Verriegelungsschraube 3 dauerhaft in der gewählten Lage winkelstabil verriegelt.

Zur Erzielung einer optimalen Fixierung kann die Hülse 6 aus einem entsprechend spreizbaren Material, zum Beispiel einem für diese Anwendung geeigneten Kunststoff bestehen bzw. ein solches aufweisen.

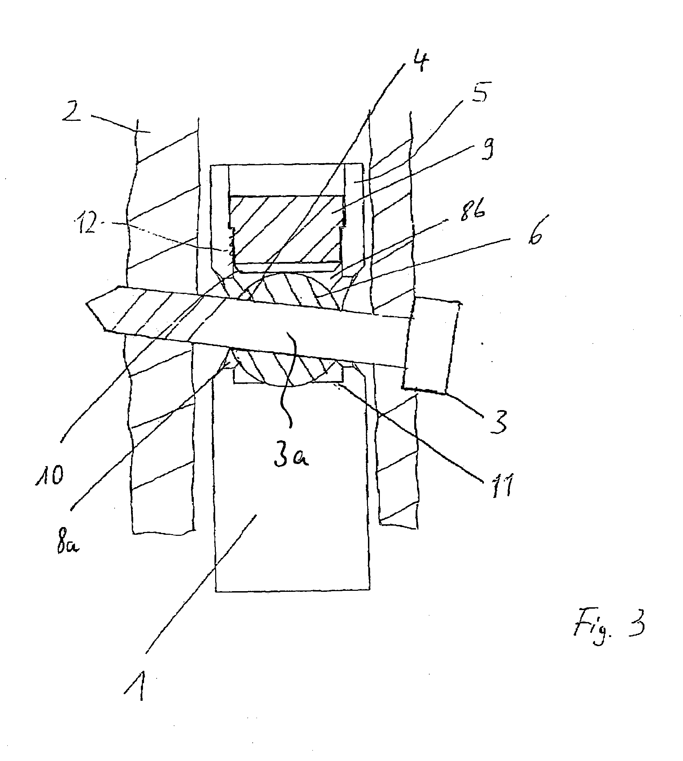

In Figur 3 ist eine andere Möglichkeit der winkelstabilen Arretierung der Hülse 6 mit der Verriegelungsschraube 3 gezeigt. Die Ausführung des Marknagels 1, der Hülse 6 und der Verriegelungsschraube 3 ist ähnlich wie in Figur 1 dargestellt. Die kugelförmige Hülse 6 kann hierbei wiederum aus einem entsprechenden Kunststoff bestehen. Die Kugel bzw. Hülse 6 liegt im Schaft 5 des Marknagels auf einer Sitzfläche 11 auf.

Oberhalb der Kugel bzw. Hülse 6 ist der Schaft 5 mit einem Gewinde 12 versehen, in das koaxial zum Schaft eine Klemmschraube 9 eingesetzt ist, die auf der Oberseite der Hülse 6 aufliegt. Durch Einschrauben der Klemmschraube 9 in das Gewinde 12 wird die Hülse auf die Sitzfläche 11 gepresst und nach dem Einbringen der Verriegelungsschraube 3 lagestabil fixiert.

Zur Erhöhung der Klemmkraft und des daraus resultierenden Lösemoments der Hülse 6 im Schaft 5 kann die Klemmschraube 9 an ihrer Kontaktfläche 10 mit der Hülse 6 entsprechend geometrisch an die Form der Hülse angepasst werden, was hier nicht dargestellt ist. Hierdurch kann die Haftreibung zwischen Klemmschraube und Hülse erhöht werden.

Zusätzlich kann die Kontaktfläche der Klemmschraube beispielsweise durch Rändeln oder das Aufbringen reibungssteigernder Beschichtungen aufgeraut werden. Eine andere Möglichkeit wäre, die Klemmschraube 9 mit einem spitz zulaufenden Ende zu versehen, welches in die Hülse 6 eindringt und diese somit zusätzlich formschlüssig fixiert.

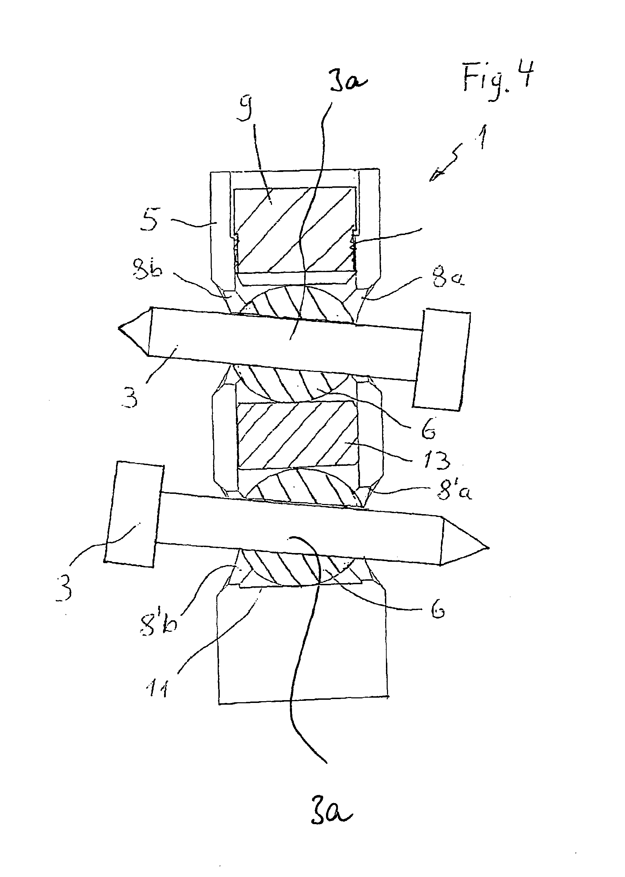

In Figur 4 ist ein Marknagel 1 mit einem Schaft 5 dargestellt, der mehrere, in diesem Falle zwei Durchbrüche 8a, 8b und 8'a, 8'b aufweist, wobei in diesen Bereichen jeweils eine Hülse 6 eingesetzt ist, in die jeweils eine Verriegelungsschraube 3 hindurch getrieben werden kann.

Zur Verriegelung der beiden Hülsen 6 ist wiederum eine Klemmschraube 9 entsprechend Figur 3 vorgesehen, die auf der oberen Hülse 6 anliegt. Zwischen den beiden Hülsen 6 ist ein blockförmiger Zwischenkörper 13 vorgesehen, der an beiden Hülsen 6 anliegt. Durch Festziehen der Klemmschraube 9 werden dann beide Hülsen 6 unter Zwischenschaltung des Zwischenkörpers 13 lagestabil arretiert

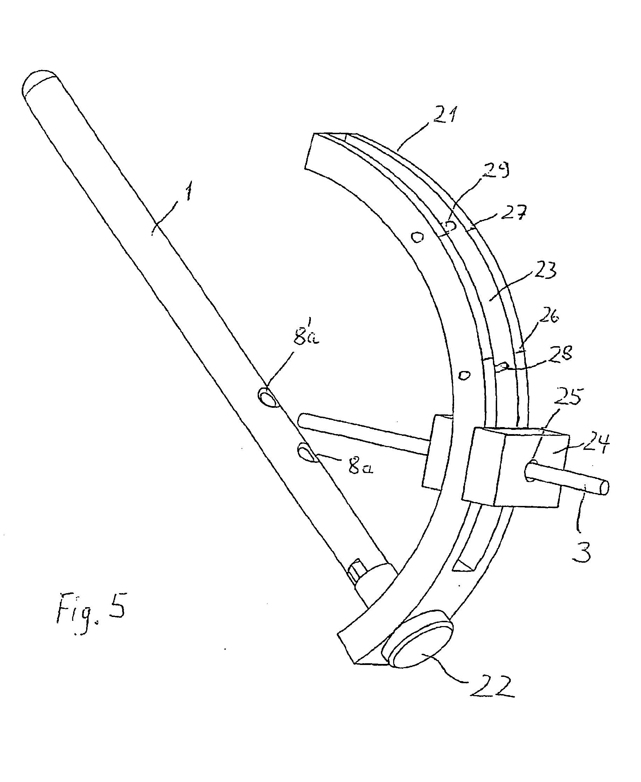

In Figur 5 ist ein Marknagel 1 gemäß der Erfindung mit zwei Durchbrüchen 8a, 8'a gezeigt, wobei dort nicht dargestellte kugelförmige Hülsen eingesetzt sind. Mit dem oberen Ende des Marknagels ist ein, nicht erfindungsgemäßer, gekrümmter, in diesem Falle ein einem Kreisbogen folgender Führungsbogen 21 mit Hilfe einer Schraube 22 lösbar verbunden, der um die Längsachse des Marknagels verschwenkbar ist und in einer gewünschten Winkelposition mit Hilfe der Schraube 22 fixiert werden kann. Der Führungsbogen 21 weist eine in seiner Längsrichtung verlaufende, im Wesentlichen mittige Führungsbahn 23 auf, die den Führungsbogen 21 durchstößt. Die Seitenwände der Führungsbahn 23 sind auf dem Marknagel 1 ausgerichtet, in der Führungsbahn 23 gleitet formschlüssig eine Schablone 24 mit einer mittigen Öffnung 25, in die eine Verriegelungsschraube 3 einsteckbar ist, wobei diese Vernegelungsschraube 3 in Richtung auf die Mittelebene des Marknagels 1 gerichtet ist. Anstelle einer Verriegelungsschraube 3 kann in die Öffnung 26 auch ein Zielrohr eingesetzt werden, in das dann erst die Verriegelungsschraube eingesetzt wird.

Vor dem Einsatz des Marknagels wird die Schablone 24 in eine erste Position verschoben, in welcher der Marknagel 1 beziehungsweise das Zielrohr auf einen Durchbruch, zum Beispiel den Durchbruch 8a gerichtet ist. Die Position der Schablone kann zum Beispiel auf dem Führungsbogen 21 durch eine Markierung 26 gekennzeichnet werden. Anschließend wird die Schablone 24 in eine zweite Position gebracht/ in der sie auf den Durchbruch 8'a gerichtet ist. Auch diese Position wird durch eine Markierung 27 auf dem Führungsbogen 21 gekennzeichnet.

Anstatt Markierungen 26 beziehungsweise 27 vorzusehen, ist es möglich, durch den Führungsbogen 8 jeweils einen stabförmigen Riegel 28 beziehungsweise 29 hindurchzutreiben, an denen die Schablone 24 in den entsprechenden Stellungen anschlägt. Hiermit kann ein Operateur die gewünschte Winkellage der in den Marknagel 1 einzuschraubenden Verriegelungsschraube 3 festlegen.

## Patentansprüche

1. Verriegelungsmarknagel (1) zum Einbringen in den Markraum eines Röhrenknochens (2) zur Versorgung von Knochenfrakturen beziehungsweise zur Versteifung von Gelenken, mit einem länglichen Schaft (5) mit mindestens einer Öffnung (8) zur Aufnahme von einer oder mehreren Verriegelungsschrauben (3), wobei im Bereich der zumindest einen Öffnung (8) eine zumindest senkrecht zu der Längsachse des Verriegelungsmarknagels (1) verschwenkbare Hülse (6) in den Schaft (5) gelagert ist, die eine Bohrung (4) zur Aufnahme einer Verriegelungsschraube (3) aufweist und nach Einsetzen der Verriegelungsschraube (3) in dem Schaft (5) lagestabil verriegelbar ist,
**dadurch gekennzeichnet, dass**
- bei wenigstens zwei aufeinander folgenden Hülsen (6) in oder am Verriegelungsmarknagel (1) zwischen den beiden Hülsen (6) ein an diesen anliegender Zwischenkörper (13) vorgesehen ist, und
- wobei die Anordnung aus den Hülsen (6) und dem Zwischenkörper (13) durch zumindest eine in Längsrichtung des Verriegelungsmarknagels (1) wirkende Klemmschraube (9) zusammenpressbar und/oder blockierbar und/ oder arretierbar ist.

2. Verriegelungsmarknagel (1) nach Anspruch 1, **dadurch gekennzeichnet, dass** die Hülsen (6) jeweils eine Walze mit einer Walzenachse senkrecht zu der Längsachse des Schafts (5) des Verriegelungsmarknagels (1) sind.

3. Verriegelungsmarknagel (1) nach Anspruch 1, **dadurch gekennzeichnet, dass** die Hülsen (6) kugelförmig sind und in mehr als einer Raumachse, insbesondere in allen Raumachsen, frei schwenkbar oder drehbar in dem Schaft (5) angeordnet, insbesondere gelagert sind.

4. Verriegelungsmarknagel (1) nach einem der vorhergehenden Ansprüche, **dadurch gekennzeichnet, dass** die Hülsen (6) durch Kraftschluss arretierbar sind, insbesondere durch das Vorsehen einer Einrichtung zum kraftschlüssigen Arretieren.

5. Verriegelungsmarknagel (1) nach einem der vorhergehenden Ansprüche, **dadurch gekennzeichnet, dass** die Hülsen (6) durch Formschluss arretierbar sind bzw. durch das Vorsehen einer Einrichtung zum formschlüssigen Arretieren.

6. Verriegelungsmarknagel (1) nach einem der vorhergehenden Anspruche, **dadurch gekennzeichnet, dass** die Hülsen (6) geschlitzt oder aufweitbar ausgeführt sind, und dass die zugehörige Verriegelungsschraube (3) zur Lagefixierung zumindest in einem Bereich konisch ausgeführt ist.

7. Verriegelungsmarknagel (1) nach einem der vorhergehenden Ansprüche, **dadurch gekennzeichnet, dass** die Lagefixierung der Hülsen (6) durch wenigstens eine zum Schaft (5) des Verriegelungsmarknagels (1) koaxial und/oder im wesentlichen parallel zur Längsachse des Verriegelungsmarknagels (1) angeordnete Klemmschraube (9) erfolgt.

8. Verriegelungsmarknagel (1) nach einem der vorhergehenden Ansprüche, **dadurch gekennzeichnet, dass** die Hülsen (6) Kunststoff aufweisen oder aus Kunststoff bestehen.

9. Verriegelungsmarknagel (1) nach einem der Ansprüche 1 bis 8, **dadurch gekennzeichnet, dass** bei mehreren aufeinander folgenden Hülsen (6) in oder am Verriegelungsmarknagels (1) wenigstens zwei der Hülsen (6) sich durch das Einwirken einer Klemmschraube (9) gegenseitig zusammenpressen oder blockieren oder arretieren.

## Claims

1. A locking intramedullary nail (1) for insertion into the medullary cavity of a hollow bone (2) and for supporting bone fractures and reinforcing or stiffening joints, respectively, comprising an elongated shaft (5) having at least one through hole (8) for receiving one or more locking screws (3), wherein in an area of the at least one through hole (8) a sleeve (6) which is pivotable at least perpendicularly to the longitudinal axis of the locking intramedullary nail (1) is supported in the shaft (5) and has a bore (4) for receiving a locking screw (3) and is lockable in a positionally stable manner after insertion of the locking screw (3) into the shaft (5),
**characterised in that**
- in the case of at least two succeeding sleeves (6) in or at the intramedullary nail (1), an intermediate element (13) abuting against the sleeves (6) is provided between the sleeves (6), and
- wherein the arrangement of sleeves (6) and intermediate element (13) is compressable and/or blockable and/or arrestable by at least one clamping screw (9) which exerts force in the longitudinal direction of the locking intramedullary nail (1).

2. Locking intramedullary nail according to claim 1, **characterised in that** each sleeve (6) is a roller having a roller axis which is perpendicular to the longitudinal axis of the shaft (5) of the locking intramedullary nail (1).

3. Locking intramedullary nail according to claim 1, **characterised in that** the sleeves (6) are spherical and are arranged, in particular supported, freely pivotably or rotatably in more than one spatial axis, particularly in all spatial axes, in the shaft (5).

4. Locking intramedullary nail according to anyone of the preceding claims, **characterised in that** the sleeves (6) are arrestable through frictional connection, in particular by means of the provision of a device for arresting by means of frictional connection.

5. Locking intramedullary nail according to anyone of the preceding claims **characterised in that** sleeves (6) are arrestable by means of form closure connection or by means of the provision of a device for arresting by means of form closure connection, respectively.

6. Locking intramedullary nail according to anyone of the preceding claims, **characterised in that** the sleeves (6) are embodied in a slit or expandable design and **in that** the related locking screw (3) has a conical design in at least one section thereof in order to achieve the positional fixation.

7. Locking intramedullary nail according to anyone of the preceding claims, **characterised in that** the positional fixation of the sleeves (6) is achieved by means of at least one clamping screw (9) which is arranged coaxially to the shaft (5) of the intramedullary nail (1) and/or substantially parallel to the longitudinal axis of the intramedullary nail (1).

8. Locking intramedullary nail according to anyone of the preceding claims, **characterised in that** the sleeves (6) comprises or consists of synthetic or plastic material.

9. Locking intramedullary nail according to anyone of the claims 1 to 8, **characterised in that** in the case of several succeeding sleeves (6) in or at the intramedullary nail (1) which are arranged in or on the locking intramedullary nail (1), at least two of the sleeves (6) compress or block or arrest each other by means of the force of the clamping screw (9).

## Revendications

1. Un clou de verrouillage intramédullaire (1) pour insertion dans le canal médullaire d'un os creux (2) pour assister une fracture osseuse ou pour rigidifier une articulation respectivement, comprenant une tige allongée (5) avec au moins un orifice (8) pour recevoir une ou plusieurs vis de verrouillage (3), où dans une zone de l'orifice (8) une gaine (6) située dans la tige (5), pivotable au moins perpendiculairement à l'axe longitudinal du clou de verrouillage intramédullaire (1) présente un trou (4) pour recevoir une vis de verrouillage (3) et est verrouillable en position stable après insertion de la vis de verrouillage (3) dans la tige (5),
**caractérisé en ce que**
- pour au moins deux gaines successives (6) dans ou au niveau du clou de verrouillage (1), un corps intermédiaire (13) contigu aux gaines est prévu entre les gaines (6), et
- où l'arrangement des gaines (6) et du corps intermédiaire (13) peut être comprimé et/ou bloqué et/ou arrêté tout au moins par une vis de serrage (9) agissant dans une direction longitudinale du clou de verrouillage intramédullaire (1).

2. Clou de verrouillage intramédullaire (1) selon la première revendication, **caractérisé en ce que** chaque gaine (6) est un cylindre ayant un axe perpendiculaire à l'axe longitudinal de la tige (5) du clou de verrouillage intramédullaire (1).

3. Clou de verrouillage intramédullaire (1) selon la première revendication, **caractérisé en ce que** les gaines (6) sont sphériques et agencées, en particulier supportées, de façon à pouvoir pivoter ou tourner librement dans au moins un axe de l'espace, en particulier dans tous les axes de l'espace.

4. Clou de verrouillage intramédullaire (1) selon l'une quelconque des revendications précédentes **caractérisé en ce que** les gaines (6) peuvent être bloquées par adhérence, en particulier par un dispositif de blocage par adhérence.

5. Clou de verrouillage intramédullaire (1) selon l'une quelconque des revendications précédentes **caractérisé en ce que** les gaines (6) peuvent être bloquées par complément de forme ou par un dispositif de blocage par complément de forme, respectivement.

6. Clou de verrouillage intramédullaire (1) selon l'une quelconque des revendications précédentes **caractérisé en ce que** les gaines (6) sont fendues ou réalisées de façon expansible et où au moins une zone de la vis de verrouillage (3) correspondante pour la fixation en position est conique.

7. Clou de verrouillage intramédullaire (1) selon l'une quelconque des revendications précédentes **caractérisé en ce que** la fixation en position des gaines (6) est obtenue au moyen d'au moins une vis de serrage (9) agencée de façon coaxiale à la tige (5) du clou de verrouillage intramédullaire (1) et/ou de façon substantiellement parallèle à l'axe longitudinal du clou de verrouillage intramédullaire (1).

8. Clou de verrouillage intramédullaire (1) selon l'une quelconque des revendications précédentes **caractérisé en ce que** les gaines (6) comprennent ou sont formées d'une matière synthétique ou plastique.

9. Clou de verrouillage intramédullaire (1) selon l'une quelconque des revendications 1 à 8 caractérisé en ce dans le cas de plusieurs gaines (6) successives agencées dans ou au niveau du clou de verrouillage (1), au moins deux des gaines (6) se compriment ou se bloquent ou s'arrêtent les unes les autres au moyen de la force de la vis de serrage (9).
